# EUROPEAN PATENT APPLICATION

(11) **EP 4 729 031 A1**
(43) Date of publication of application: **22.04.2026**
(21) Application number: 25209326.5
(22) Date of filing: 17.10.2025
(51) Int. Cl.: A61F 2/38

(54) **FEMORAL IMPLANT WITH LATERALLY ROTATED TROCHLEA**

(30) Priority: 17.10.2024 US 202463708379 P
(71) Applicant: Howmedica Osteonics Corp., Mahwah, NJ 07430 (US)
(72) Inventor: Crutcher, James, Seattle, 98112 (US)
(74) Representative: Keller Schneider Patentanwaltsgesellschaft mbH

(57) **Abstract**

A femoral implant includes a medial compartment, a lateral compartment and a trochlear groove in between the medial compartment and the lateral compartment. A central axis bifurcates the femoral implant and extends in a transverse plane perpendicular to a posterior condylar axis, the posterior condylar axis passing through a posterior-most point of the medial compartment and a posterior-most point of the lateral compartment. The trochlear groove is aligned on a trochlear groove axis that extends from the posterior condylar axis at a lateral angle relative to the central axis, the trochlear groove axis being in the transverse plane.

## Description

### CROSS-REFERENCE TO RELATED APPLICATION

The present application claims the benefit of the filing date of U.S. Provisional Patent Application No. 63/708,379 filed October 17, 2024, the disclosure of which is hereby incorporated by reference herein in its entirety.

### BACKGROUND

Mechanical alignment has been widely used in Total Knee Arthroplasty ("TKA") during the past several decades. It is a fairly reproducible technique when used with manual instruments that are limited to two-dimensional line of sight orientation. Up to the recent adoption of robotic surgery, it was the preferred method of alignment. With the increased adoption of robotic surgery and 3D planning, implant alignment has increasingly shifted away from mechanical into a more kinematically based implant orientation, aptly named kinematic alignment.

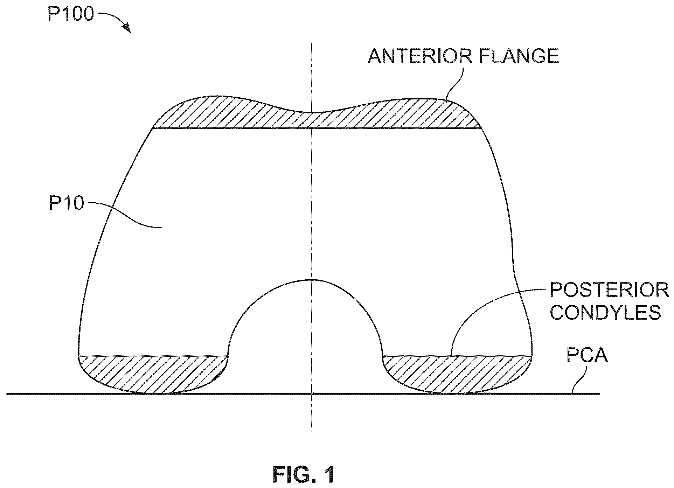

In mechanical alignment, the tibia is resected perpendicular to the mechanical axis of the tibia creating a resection depth that is lesser medially than it is laterally since the natural tibia is typically in about 3 degrees of varus. In order to create cuts on the femur that are parallel to the tibial resection, a compensatory adjustment has to be made on the femur by externally rotating the femoral implant, resulting in more bone resected medially than laterally with respect to the Posterior Condylar Axis ("PCA") of the femur. In kinematic alignment, the femoral implant, such as the conventional femoral implant P100 shown in cross-section implanted on a femur P10 in FIG. 1, is generally aligned with the PCA (in FIG. 1, PCA P30) or, if not exactly aligned with the PCA, is still internally rotated to some extent with respect to the position of the femoral implant if it were placed in mechanical alignment. However, when the femoral implant is aligned in this way, i.e., kinematically, poor patella-femoral interaction may result and the clinical outcome may be negatively impacted.

### BRIEF SUMMARY

In some aspects, the present disclosure contemplates a femoral implant with an improved trochlear alignment in the transverse plane. An advantage of the femoral implant according to these aspects is that it may be implanted in kinematic alignment with the femur bone of a patient while also preserving congruity with the natural trochlear alignment of the femur bone. In this manner, when such femoral implant is implanted in a patient, the trochlea of the femoral implant is anatomically positioned. This improvement is realized through an increase in the lateral obliquity of the trochlea with respect to the PCA of the femoral implant. Specifically, the trochlea of the femoral implant is obliquely oriented with respect to the PCA in a laterally rotated direction. Put another way, a trough in the femoral implant surface that forms the trochlear groove is aligned, as viewed in the transverse plane, such that the alignment is externally rotated relative to a central axis, the central axis being perpendicular to the PCA. The alignment of the trochlea may be such that the trochlea is laterally rotated relative to a centralized alignment by an angle in a range from 3 degrees to 8 degrees. For instance, in some non-limiting examples, the trochlea may be laterally rotated relative to a central alignment by an angle of 3.5 degrees, 4.0 degrees, 4.5 degrees, 5.0 degrees, 5.5 degrees, 6.0 degrees, 6.5 degrees, 7.0 degrees or 7.5 degrees. In the above description, the central axis bifurcates the femoral component in the transverse plane and is perpendicular to the PCA.

Examples of femoral implants will now be described. It will be appreciated that the described examples summarize one or more aspects of the present disclosure as provided in the detailed description and the figures. Accordingly, the examples are also to be read in connection with the detailed description and the appended figures, and do not limit the present disclosure. The features and preferences as described elsewhere in the present disclosure also apply to the following examples.

In a first aspect, a femoral implant includes an anterior portion with medial and lateral condylar portions extending therefrom. A trochlear groove is defined within the anterior portion and the medial and lateral condylar portions. The trochlear groove is shaped such that when viewed in a transverse plane, an alignment axis of the trochlear groove over a portion of the trochlear groove between a posterior extent of the femoral implant and an anterior extent of the femoral implant is at a lateral angle relative to a central implant axis orthogonal to an implant posterior condylar axis. In the above-described arrangement, the alignment axis and the central implant axis are in or parallel to the transverse plane.

In some examples of the first aspect, the angle may be in a range from 3 degrees to 8 degrees lateral to the central axis. In a subset of these examples, the angle may be in a range from 3 degrees to 6 degrees lateral to the central axis. In some examples, a maximum depth of the trochlear groove may be coincident with the alignment axis of the trochlear groove. In some examples, the trochlear groove may be partially defined along a sub portion of the portion where there is an absence of material along the alignment axis. In some examples, the posterior extent may be one of a medial posterior peak of the medial condyle and a lateral posterior peak of the lateral condyle and the anterior extent may be one of a medial anterior peak of the anterior portion and a lateral anterior peak of the anterior portion. In some examples, the implant posterior condylar axis may pass through the medial posterior peak and the lateral posterior peak. In yet another example, the femoral implant of the first aspect may be used in a method of implanting the femoral implant. The method may include a step of aligning the femoral implant on a resected surface of a distal femur of a patient such that the implant posterior condylar axis is aligned with a posterior condylar axis of the distal femur. In some examples of the method, the femoral implant may be fixed to the femur using bone cement.

In a second aspect, a femoral implant includes an articular surface extending from an anterior side to a posterior side, the articular surface including a medial portion and a lateral portion. The femoral implant also includes a trochlear groove surface in between the medial portion and the lateral portion. The femoral implant includes an implant posterior condylar axis that passes through a medial posterior peak on the articular surface and a lateral posterior peak on the articular surface, the medial and lateral posterior peaks being at a posterior-most extent of the respective medial and lateral portions. Further, when the femoral implant is viewed in a transverse plane, an alignment axis passing through a plurality of low points of the trochlear groove surface is at an acute angle relative to the implant posterior condylar axis, the acute angle being lateral relative to a plane that bifurcates the femoral implant when viewed in the transverse plane. Put another way, the trochlear groove surface may extend anterolaterally from the posterior side toward the anterior side.

In some examples of the second aspect, the acute angle may be in a range from 82 degrees to 87 degrees. In a subset of these examples, the acute angle may be in a range from 86 degrees to 87 degrees. In some examples, the plurality of low points may all be located closer to an anterior-most extent of the articular surface than the posterior-most extent in the transverse plane. In some examples, an anterior-most extent of the medial compartment is further from the implant posterior condylar axis than an anterior-most point of the lateral compartment. In some examples, an axis through the anterior-most point of the medial compartment and the anterior-most point of the lateral compartment is at an angle relative to the implant posterior condylar axis that, when added to the acute angle, totals ninety degrees.

In a third aspect, a femoral implant includes a body including a trochlear groove separating a medial condyle and a lateral condyle. The trochlear groove is configured such that, when viewed in a transverse plane, the trochlear groove defines a path in the body that is at a lateral angle relative a central axis in the transverse plane, the central axis being perpendicular to a posterior condylar axis of the femoral implant. In some examples of the third aspect, the lateral angle may be in a range from 3 degrees to 8 degrees. In a subset of these examples, the lateral angle may be in a range from 3 degrees to 4 degrees. In some examples, the path of the trochlear groove in the body may be centered along a low point on an articular surface of the body. In some examples, an anterior-most point on the medial condyle may be further from the posterior condylar axis in the transverse plane than an anterior-most point on the lateral condyle.

In a fourth aspect, a femoral implant includes a medial compartment, a lateral compartment and a trochlear groove in between the medial compartment and the lateral compartment. A central axis bifurcates the femoral implant and extends in a transverse plane perpendicular to a posterior condylar axis, the posterior condylar axis passing through a posterior-most point of the medial compartment and a posterior-most point of the lateral compartment. The trochlear groove is aligned on a trochlear groove axis that extends from the posterior condylar axis at a lateral angle relative to the central axis. And, the trochlear groove axis is in the transverse plane.

In some examples of the fourth aspect, the lateral angle may be in a range from 3 degrees lateral to the central axis to 8 degrees lateral to the central axis. In some examples, the lateral angle may be in a range from 3 degrees lateral to the central axis to 4 degrees lateral to the central axis. In some examples, a maximum depth of the trochlear groove (relative to distal most points of the condyles) may be coincident with the trochlear groove axis of the trochlear groove. Put another way, the maximum depth of the trochlear groove along a length of the trochlear groove may be parallel with the trochlear groove axis of the trochlear groove. In some examples, the medial compartment has an anterior-most point in the transverse plane and the lateral compartment has an anterior-most point in the transverse plane, and the anterior-most point of the medial compartment may be further from the posterior condylar axis than the anterior-most point of the lateral compartment. In a subset of the immediately preceding examples, an anterior axis passes through the anterior-most point of the medial compartment and the anterior-most point of the lateral compartment in the transverse plane, and an angle between the anterior axis and the posterior condylar axis may be the same as the lateral angle.

In still further examples of the fourth aspect, a path of the trochlear groove may be centered along a low point between the medial compartment and the lateral compartment. In other examples, the transverse plane may be 15 mm proximal to a distal-most point on the medial compartment.

In some aspects, the present disclosure relates to methods of implanting a femoral implant on a femur. In a fifth aspect, a method of implanting a femoral implant on a femur may be performed through steps including: positioning a femoral implant on a resected surface on a distal end region of a femur of a patient such that posterior-most extents of medial and lateral condyles of the femoral implant are aligned with a posterior condylar axis of the femur and, in a transverse plane, a trochlear groove of the femoral implant is at a lateral angle relative to a central axis of the femoral implant, the central axis being perpendicular to the posterior condylar axis; and fixing the femoral implant in place relative to the femur. In some examples, fixation of the femoral implant may include the use of bone cement. In other examples, the femoral implant may include surface features to facilitate cementless fixation.

In some examples of the fifth aspect, the method may include the following steps: with a knee joint of the patient in flexion, resecting the femur along a first plane parallel to the posterior condylar axis so as to remove posterior portions of the medial condyle and the lateral condyle; and with the knee joint of the patient in extension, resecting the femur along a second plane spaced apart from a distal end of the femur so as to remove a distal portion of the femur.

In the above or other examples, the method may include the following step: when the femoral implant is fixed in place relative to the femur, a patella of the patient is stable within the trochlear groove through a range of motion of a knee joint of the patient where the range of motion extends from flexion to extension.

### BRIEF DESCRIPTION OF THE DRAWINGS

FIG. 1 is a cross-sectional view in a transverse plane of a conventional femoral implant;
FIG. 2A is a cross-sectional view in a sagittal plane of a distal femur with a femoral implant modeled thereon;
FIG. 2B is a cross-sectional view in a transverse plane of the distal femur of FIG. 2A.
FIG. 3 is a cross-sectional view in a transverse plane of a distal femur with a femoral implant modeled thereon;
FIG. 4 is a cross-sectional view in a transverse plane of a distal femur with a femoral implant modeled thereon;
FIG. 5 is a cross-sectional view of a femur for planning a femoral implant design according to one embodiment of the present disclosure;
FIG. 6 is a bottom view of a femoral implant according to one embodiment of the present disclosure;
FIGs. 7 and 8 illustrate steps in a method of preparing a femur for implantation of a femoral implant, according to one embodiment of the present disclosure; and
FIG. 9 illustrates another step in the method of FIGs. 7 and 8 where a femoral implant is implanted in kinematic alignment on the femur.

### DETAILED DESCRIPTION

As used herein, the term "distal" means more distant from the heart and the term "proximal" means closer to the heart. The term "inferior" means toward the feet and the term "superior" means towards the head. The term "anterior" means towards the front part of the body or the face and the term "posterior" means towards the back of the body. The term "medial" means toward the midline of the body and the term "lateral" means away from the midline of the body.

As used herein, the terms "substantially," "generally," "approximately," and "about" are intended to mean that slight deviations from absolute are included within the scope of the term so modified.

In the present disclosure, a femoral implant, also referred to as a femoral component, has been developed that addresses the problem of poor matching of femoral implants with femurs at a distal end of the femur as part of total knee arthroplasty (hereinafter "TKA"). Specifically, a femoral implant with a trochlear groove as contemplated by embodiments of the present disclosure has been found to dramatically increase the instances where a femoral implant successfully matches a patient's anatomy, particularly when accounting for any rotational adjustment in the transverse plane deemed appropriate to account for kinematics of the knee and ligament balancing, e.g., positioning the femoral implant with an internal rotation to align with the PCA of the femur bone.

In one aspect, the present disclosure relates to a femoral implant configured for implantation on a distal femur in a knee joint. The femoral implant includes a trochlear groove shaped such that when viewed in a transverse plane, i.e., looking along a sight line parallel to that of a length of the femur, a path of the trochlear groove extends in a direction that is laterally angulated relative to a centerline of the femoral implant that extends in an anterior-posterior direction.

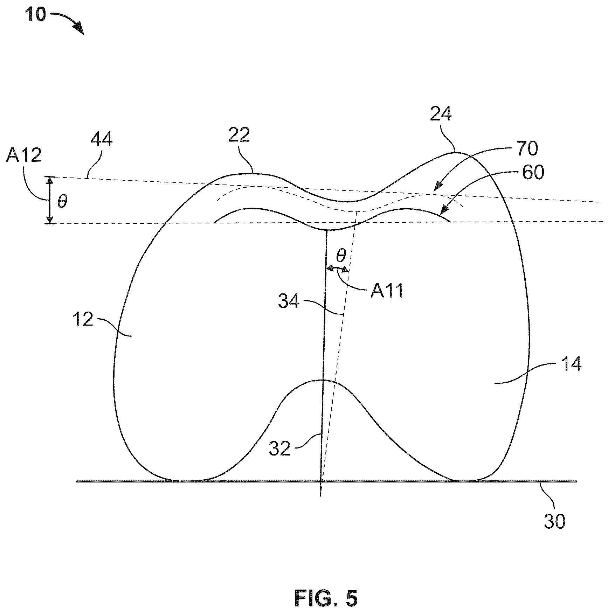

A view in the transverse plane that illustrates how a shape of the contemplated femoral implant is determined is shown in FIG. 5. In FIG. 5, a distal femur 10 viewed in a transverse plane includes a medial condyle 12 and a lateral condyle 14, with a central axis 32 separating the condyles that extends perpendicularly relative to a posterior-condylar axis 30 of the femur. A shape of a trochlea, or trochlear groove is established by reorienting the trochlea through an increase in lateral obliquity relative to central axis 32. In FIG. 5 specifically, a difference in orientation between a mechanical alignment 60 and a kinematic alignment 70 is shown. As will be described in greater detail elsewhere in the present application, orienting the trochlea at a lateral angle A11 relative to the central axis of the femoral implant allows for the trochlear groove to be congruent, or at least to have increased congruency with respect to the native trochlear groove of a femur of a patient when the implant is positioned on the femur in a kinematic alignment 70. Specifically, a femoral implant with a trochlea having an alignment axis 34 in a range from 3 degrees to 6 degrees laterally rotated relative to a central axis 32 of the femoral implant significantly improves anatomical matching of the trochlear groove in TKA. This lateral rotation of the trochlear groove relative to the central axis ensures that the trochlea of the femoral implant remains in a more natural alignment when the femoral implant is internally rotated to align with the PCA of a native femur anatomy (i.e., for kinematic alignment). Greater congruity of the implant trochlea with the native trochlea ensures that the implant trochlea maintains its functional role in patellar tracking and knee articulation without being compromised by the internal rotation of the femoral implant overall, thus providing a stable implant with the benefits of kinematic alignment, such as improved balance of the flexion gap in the knee.

Additionally, and with continued reference to the femur in the transverse plane as shown in FIG. 5, the femur has a medial anterior eminence 22 and a lateral anterior eminence 24 with an axis passing through both. The eminences may also be referred to as peaks or anterior-most points. In some example implementations, when a femoral implant includes a trochlea oriented at a lateral angle A11, an axis 44 passing through the medial anterior eminence and the lateral anterior eminence of the femoral implant may be at an angle A12 relative to the PCA 30, where the angle A12 is the same or similar to angle A11, as shown in FIG. 5. Stated another way, axis 44 may be perpendicular, or nearly perpendicular to alignment axis 34.

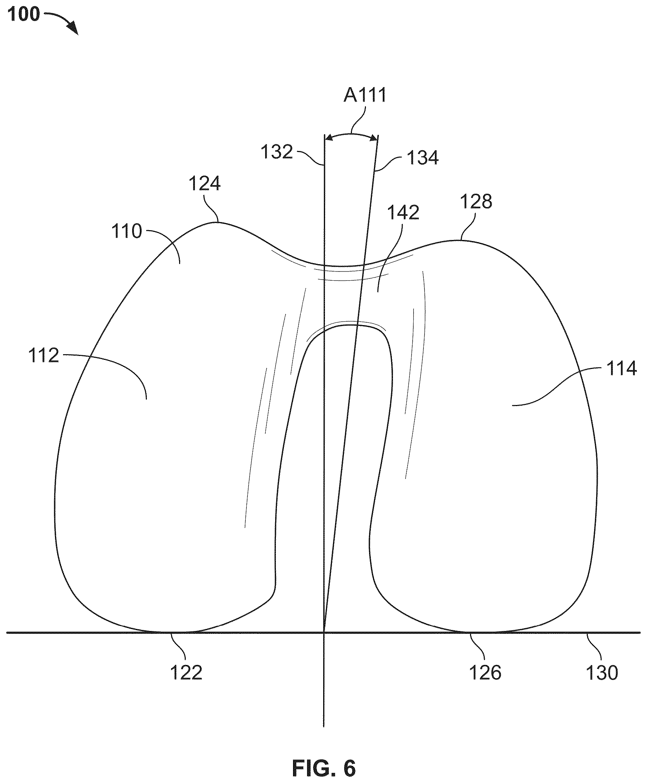

In some examples, a femoral implant may be as shown in FIG. 6 and includes an anterior flange and medial and lateral condyles extending therefrom. A trochlear groove separates the medial and lateral condyles and extends from a posterior end toward the anterior flange. In the transverse plane, an alignment of the trochlear groove is at a lateral angle relative to a central axis of the femoral implant, where the central axis is perpendicular to an implant posterior condylar axis, implant PCA 130. It should be appreciated that FIG. 6 is provided for illustrative purposes only and the contours of the depicted femoral implant may differ from what would actually be included in a manufactured implant.

Femoral implant 100 is shown in a transverse plane in FIG. 6 and includes an anterior portion 110, a medial condyle 112, which is part of a medial compartment and extends from the anterior portion, and a lateral condyle 114, which is part of a lateral compartment and also extends from the anterior portion. Trochlear groove 142 extends centrally in a generally posterior-anterior direction in the transverse plane. The implant PCA 130 extends through a medial posterior peak 122 of medial condyle 112 and a lateral posterior peak 126 of lateral condyle 114. On an anterior-most extent of femoral implant 100 in the transverse plane are medial anterior peak 124 and lateral anterior peak 128. Central axis 132 of femoral implant 100 is perpendicular to implant PCA 130. Both implant PCA 130 and central axis 132 are in the transverse plane or may also be described as being parallel to the transverse plane. In some examples, central axis 132 bifurcates femoral implant 100 in the transverse plane.

An alignment axis 134 of trochlear groove 142 is shown in FIG. 6. Alignment axis 134 is laterally angled relative to central axis 132 by an angle of A111. The alignment axis 134 extends away from implant PCA 130 in an anterolateral direction. In some examples, an angle between central axis 132 and alignment axis 134 may be in a range from 3 degrees to 8 degrees. In a subset of these examples, the angle may be in a range from 3 degrees to 6 degrees. In some non-limiting examples, the trochlea may be laterally rotated relative to a central axis 132 in the transverse plane by an angle of 3.5 degrees, 4.0 degrees, 4.5 degrees, 5.0 degrees, 5.5 degrees, 6.0 degrees, 6.5 degrees, 7.0 degrees or 7.5 degrees. Lateral angles within this range have been found to provide a universal fit among a significant variety of femur shapes (e.g., among different sizes of femurs, different shapes of femurs and different demographic profiles) and/or kinematic requirements.

The referenced alignment of the trochlear groove 142 extends over a portion of the trochlear groove as visible in the transverse plane and generally extends over a distance from a posterior-most extent of femoral implant 100 to an anterior-most extent of femoral implant 100 in the transverse plane, or over a subset of that distance. In some examples, an alignment of the trochlear groove is commensurate with a low line of a concave dip in an articular, i.e. joint facing surface of the femoral implant. Low points on the low line may also be referred to as a maximum depth of the trochlear groove. An orientation of alignment axis 134 relative to central axis 132 is such that femoral implant 100 has lateral obliquity that is absent from a femoral implant with a symmetrical alignment. In this manner, the trochlear groove of the femoral implant more closely adheres to an anatomic trochlear groove alignment even when the implant is positioned on the distal femur with some internal rotation to accommodate kinematic alignment, i.e., when the posterior condyles of the femoral implant are aligned with the PCA of the femur.

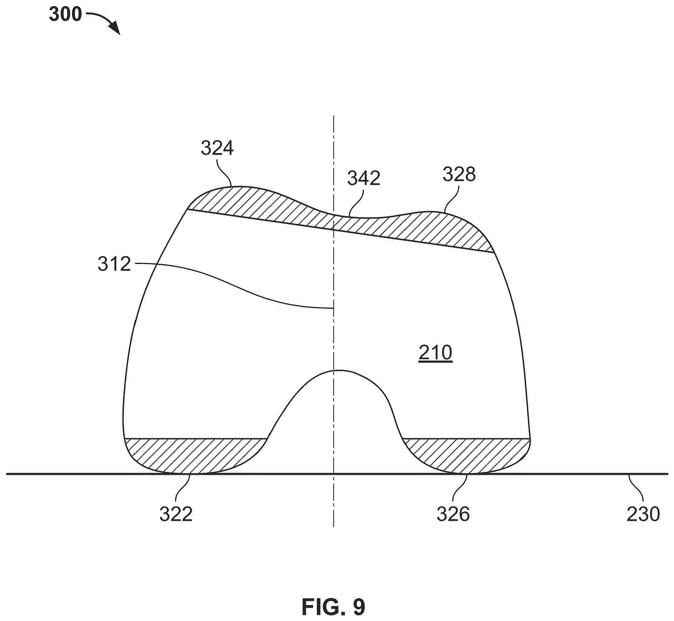

In other examples, a femoral implant may have one or more features as described for femoral implant 100, but may have a different outer profile. For example, a central opening between the condyles may be different. Such differences may include a size of the opening and/or its contours. In a subset of these examples, there may be minimal opening space between the condyles. Another example of a femoral implant that includes a laterally angled trochlea is shown in cross-section in FIG. 9 (the cross-section being through the bone in the transverse plane). The example of FIG. 9 is described in greater detail in the description of the methods of implanting a femoral implant according to other aspects of the present disclosure. Femoral implants of the present disclosure may be made of a cobalt-chromium alloy, a titanium alloy, another alloy, or another biocompatible metallic material or combination of materials suitable for knee implantation.

In another aspect, the present disclosure relates to a kit including two or more femoral implants. A femoral implant included in a kit may be femoral implant 100, femoral implant 300, or any other femoral implant contemplated by the present disclosure. In some examples, a kit may include at least one implant of a first size and at least one implant of a second size different from the first size. The size of the implant may be based on an anterior-posterior dimension of the implant, a medial-lateral dimension of the implant, a combination of both, or other dimensions of the implant. In examples of kits with femoral implants of different sizes, an alignment axis of a trochlear groove of the respective femoral implants may be at a lateral angle relative to a central axis in the transverse plane, where the central axis is perpendicular to a posterior condylar axis of the femoral implants. In a subset of these examples, the lateral angle for each femoral implant may be in a range of3 degrees to 8 degrees. In a further subset of these examples, the lateral angle for each femoral implant may be in a range from 3 degrees to 4 degrees.

In another aspect, the present disclosure relates to a method of preparing a knee for receipt of a femoral implant, including resection of the femur, and implanting a femoral implant on the resected femur of the knee.

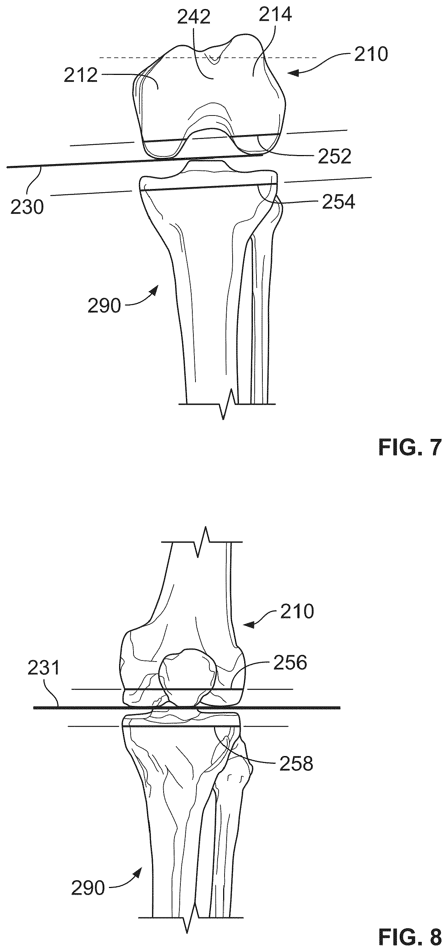

In one embodiment, a method of implanting a femoral implant is as shown in FIGs. 7-9. The method may begin with preparation of the knee to receive the femoral implant. In some variations of the method, the method may begin with the knee already resected. When bone preparation is performed, it may include resection of the femur 210 and tibia 290 as shown in FIGs. 7-8. This resection is performed to prepare the bone surfaces for receipt of an implant in kinematic alignment and includes resections with the knee in flexion, as shown in FIG. 7 and with the knee in extension, as shown in FIG. 8. In flexion, a posterior side of the femur is cut along a plane 252 that is parallel to PCA 230, with a parallel cut being made on the tibia along plane 254. Cutting femur along plane 252 ensures that a medial condyle 212 and lateral condyle 214 of femur 210 are cut to the same extent, and allows for placement of a femoral implant on the femur in a kinematic alignment. In extension, a distal end of the femur is cut along plane 256, parallel to the joint line 231, and a parallel cut plane 258 guides a cut of the tibia 290.

With all cuts of the femur and tibia are completed, a femoral implant with a laterally angulated trochlea, such as femoral implant 300 shown in FIG. 9, may be implanted on femur 210. To the extent not otherwise described, like reference numerals in the 300-series of numerals refer to like elements in the 100-series of numerals in FIG. 6. Because the posterior cuts on the femur 210 are parallel to the PCA 230, femoral implant 300 may be positioned on the distal femur so that medial posterior peak 322 and lateral posterior peak 326 are on the PCA 230 and femoral implant 300 provides kinematic alignment for the knee. With femoral implant 300 positioned on the femur aligned with the PCA 230, femoral implant 300 is typically rotated internally somewhat relative to a rotational position of a femoral implant that would be implanted on a distal femur in mechanical alignment. However, because femoral implant 300 has a laterally angulated trochlea 342, i.e. trochlear groove (relative to a central axis 312 in the transverse plane), the internal rotation of the overall implant is compensated for by the lateral angulation of the trochlea, and the congruency of the trochlea 342 of femoral implant 300 with the trochlea 242 of femur 210 is preserved. Put another way, this lateral angulation ensures that, even when the femoral implant is positioned in a kinematic alignment (i.e., internally rotated to be parallel with the PCA 230), the trochlear groove 342 remains in an anatomically accurate position. Thus, femoral implant 300 provides improved functional outcomes by better accommodating the natural biomechanics of the knee, particularly during flexion and extension, while aligning the posterior condyles in a way that improves fit and joint performance. Moreover, the lateral rotation of the trochlear groove and anterior flange of femoral implant 300 ensures a more anatomically accurate positioning, even when the femoral implant is aligned kinematically when implanted in a femur.

The above-described advantages of femoral implant 300 are also realized with the use of femoral implant 100, where the alignment axis 134 of trochlear groove 142 is angulated laterally relative to the implant posterior condylar axis 130. It should also be appreciated that the principles illustrated through the described embodiments may be applied to implants with shapes and contours that may vary from those shown in FIGs. 6 and 9, and that such variations are contemplated by the present disclosure.

### STUDY

A study was conducted that involved a review of CT-based three-dimensional pre-operative TKA plans for 220 consecutive patients. For the review of each TKA plan, a standardized axial view (i.e., view in a transverse plane) of the distal femur 15 mm proximal to the distal medial femoral condyle was used to measure the PCA of the distal femur, as well as the degree of femoral implant rotation required to improve trochlear congruity, both relative to the trans-epicondylar axis (TEA) of the femur, in all cases. A score was tabulated for each TKA plan, where the score was determined by the sum of (i) and (ii) where (i) is an angle between the PCA and TEA and (ii) is an angle of femoral component rotation relative to the TEA that would align the implant trochlea with the native trochlea in the axial plane. More specifically, (ii) is a difference in angle between a line perpendicular to the TEA and a line perpendicular to the transverse axis of the femoral component when the femoral component is rotated to achieve optimal or maximal congruity with the native trochlea in the axial plane.

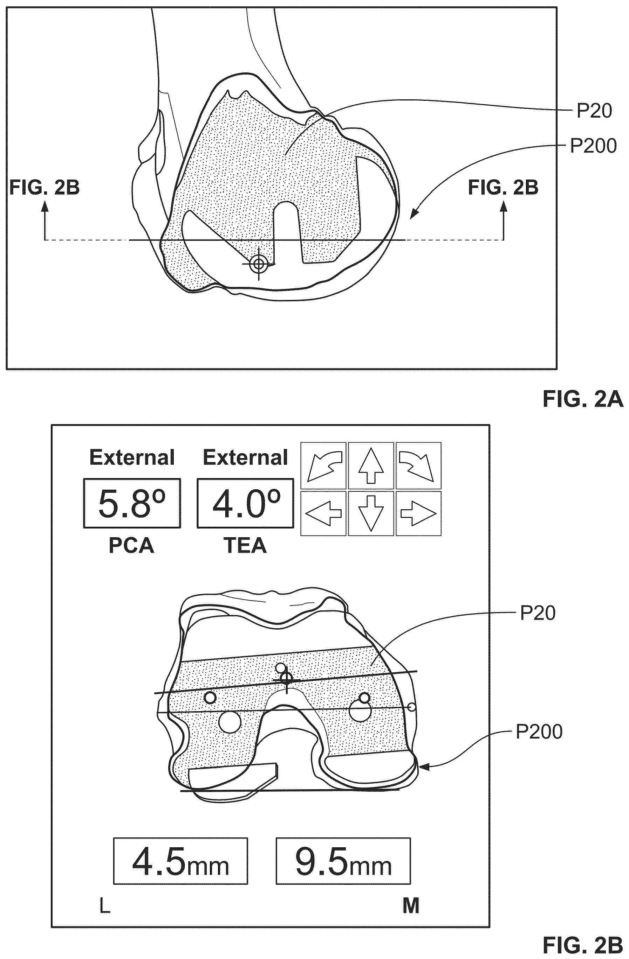

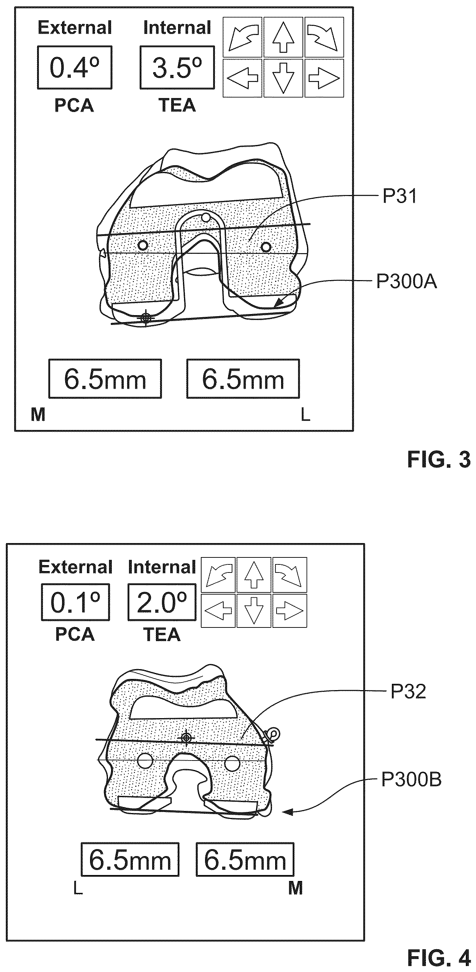

A good match was defined as a score of less than 4 °, a moderate match as a score of 4-6 °, and a poor match as a score greater than 6°. The matching scores were also sorted by gender and pre-operative limb alignment. An example of one reviewed TKA plan is shown in FIGs. 2A and 2B. FIG. 2A is a sectional view in the sagittal plane indicating that the axial view of the femur P20 with a femoral implant P200 overlaid thereon is approximately 15 mm proximal to the distal medial femoral condyle. It is at this level within the femur that the data on the trochlear groove obliquity was collected. The 15 mm depth is meaningful in that it is about half a distance along a superior-inferior direction of the trochlea and was found to be useful as a means of developing a standardized design. Additionally, the patella engages the femur at approximately this level along the femur. FIG. 2B illustrates comparative data on the PCA, the TEA and axial rotation for the TKA plan of FIG. 2A. In the TKA plan shown in FIGs. 2A and 2B, the femoral implant P200 is rotated to align the trochlea of the femoral implant P200 with the trochlea of the native bone of the femur P20. Additional TKA plans from the study are shown in FIGs. 3 and 4, where femoral implants P300A, P300B were kinematically aligned with PCAs of respective right and left knees, and the trochlea of such femoral implants was compared to the trochlear anatomy of the native femurs P31, P32, which revealed that such femoral implants lacked optimal congruity.

There was a good match (a score of less than 4 °) in 107 (49%) cases; a moderate match (a score of 4-6°) in 55 (25%) cases; and a poor match (a score of greater than 6 °) in 58 (26%) cases. In 23 (10%) cases, the mismatch was greater than 8 °. There was some disparity when the results were sorted by gender, with 21 males (19%) having a poor match, as compared to 37 females having a poor match (34%). A good match occurred in 46% of varus knees, 52% of valgus knees, and 81% of neutral alignment (+/- 2°) knees.
This CT-based study found significant variation in matching the PCA and optimal trochlear congruity when evaluating a three-dimensional pre-operative plan in a standardized axial view, i.e., in the transverse plane. As noted above, a poor match was identified in 26% of the study group. With the femoral implant of the present disclosure, the challenges associated with such lack of congruity when pursuing an anatomical alignment may be remedied.

It is to be understood that the disclosure set forth herein includes any possible combinations of the particular features set forth above, whether specifically disclosed herein or not. For example, where a particular feature is disclosed in the context of a particular aspect, embodiment, arrangement, or configuration, that feature can also be used to the extent possible, in combination with and/or in the context of other particular aspects, embodiments, arrangements, and configurations of the technology, and in the technology in general.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure.

## Claims

1. A femoral implant comprising:
a medial compartment;
a lateral compartment; and
a trochlear groove in between the medial compartment and the lateral compartment,
wherein a central axis bifurcates the femoral implant and extends in a transverse plane perpendicular to a posterior condylar axis, the posterior condylar axis passing through a posterior-most point of the medial compartment and a posterior-most point of the lateral compartment, and
wherein the trochlear groove is aligned on a trochlear groove axis that extends from the posterior condylar axis at a lateral angle relative to the central axis, the trochlear groove axis being in the transverse plane.

2. The femoral implant of claim 1, wherein the lateral angle is in a range from 3 degrees to 8 degrees lateral to the central axis.

3. The femoral implant of claim 1, wherein the lateral angle is in a range from 3 degrees to 4 degrees lateral to the central axis.

4. The femoral implant of any one of claims 1-3, wherein a maximum depth of the trochlear groove along a length of the trochlear groove is parallel with the trochlear groove axis of the trochlear groove.

5. The femoral implant of any one of claims 1-4, wherein the medial compartment has an anterior-most point in the transverse plane and the lateral compartment has an anterior-most point in the transverse plane, the anterior-most point of the medial compartment being further from the posterior condylar axis than the anterior-most point of the lateral compartment.

6. The femoral implant of claim 5, wherein an anterior axis passes through the anterior-most point of the medial compartment and the anterior-most point of the lateral compartment in the transverse plane, an angle between the anterior axis and the posterior condylar axis being the same as the lateral angle.

7. The femoral implant of any one of claims 1-6, wherein a path of the trochlear groove is centered along a low point between the medial compartment and the lateral compartment.

8. The femoral implant of any one of claims 1-7, wherein the transverse plane is 15 mm proximal to a distal-most point on the medial compartment.
